Europäisches Patentamt

(19) European Patent Office

Office européen des brevets

(11) **EP 1 457 476 B1**

(12) **EUROPÄISCHE PATENTSCHRIFT**

(45) Veröffentlichungstag und Bekanntmachung des Hinweises auf die Patenterteilung:
**22.11.2006 Patentblatt 2006/47**

(51) Int Cl.:
*C07C 5/05* [(2006.01)]     *C07C 13/275* [(2006.01)]

(21) Anmeldenummer: **03005208.8**

(22) Anmeldetag: **08.03.2003**

(54) **Selktivhydrierung von cyclododecatrien zu cyclododecen**

Selective hydrogenation of cyclododecatriene to cyclododecene

Hydrogénation sélective de cyclododecatriène à cyclododécène

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**15.09.2004 Patentblatt 2004/38**

(73) Patentinhaber: **Degussa AG**
**40474 Düsseldorf (DE)**

(72) Erfinder:
• **Herwig, Jürgen, Dr.**
**46569 Hünxe (DE)**
• **Wilczok, Norbert**
**45481 Mülheim (DE)**
• **Roos, Martin, Dr.**
**45721 Haltern am See (DE)**
• **Burghardt, Rudolf, Dr.**
**45657 Recklinghausen (DE)**
• **Gaube, Johann, Prof. Dr.**
**64380 Rossdorf (DE)**
• **Oenbrink, Georg, Dr.**
**48249 Dülmen (DE)**
• **Günzel, Bernd, Dr.**
**45721 Haltern am See (DE)**

(56) Entgegenhaltungen:
• **WIESSMEIER, GEORG ET AL: "Heterogeneously Catalyzed Gas - Phase Hydrogenation of cis, trans,trans-1,5,9-Cyclododecatriene on Palladium Catalysts Having Regular Pore Systems" INDUSTRIAL & ENGINEERING CHEMISTRY RESEARCH , Bd. 35, Nr. 12, 1996, Seiten 4412-4416, XP002246783**
• **WIESSMEIER G ET AL: "HETEROGEN KATALYSIERTE SELEKTIVHYDRIERUNG VON CIS,TRANS,TRANS- 1,5,9-CYCLODODECATRIEN AN EINEM PD/AL2O3-SCHALENKATALYSATOR" CHEMIE. INGENIEUR. TECHNIK, VERLAG CHEMIE GMBH. WEINHEIM, DE, Bd. 67, Nr. 1, 1995, Seiten 78-80, XP000485565 ISSN: 0009-286X**
• **M. BAERNS: "Lehrbuch der technischen Chemie" 1999, G. THIEME VERLAG , STUTTGART * Seite 330 - Seite 343 ***

## Beschreibung

Die vorliegende Erfindung bezieht sich auf ein Verfahren zur selektiven Gasphasenhydrierung von Cyclododecatrien und/oder Cyclododecadien zu Cyclododecen an einem festen Katalysator mit einem katalytisch aktiven Metall der Gruppe VIII des Periodischen Systems der Elemente.

Die Selektivhydrierung von Cyclododecatrien zu Cyclododecen ist in der Literatur häufig beschrieben, dabei sind zahlreiche Versuche unternommen worden, diese Selektivhydrierung mit hohem Umsatzgrad und hoher Selektivität auszuführen.

Nach allgemeiner Auffassung verläuft die Hydrierung des überwiegend als cis,trans,trans-1,5,9-Cyclododecatriens vorliegenden Cyclododecatriens (im Folgenden als CDT bezeichnet) stufenweise über Diene, vorwiegend trans, trans-1,5-Cyclododecadien und cis,trans-1,5-Cyclododecadien (beide im Folgenden als CDD bezeichnet) und die nahezu dem Gleichgewicht entsprechende Isomerenmischung trans- und cis-Cyclododecen (im Folgenden als CDEN bezeichnet) zum Cyclododecan (im Folgenden als CDAN bezeichnet).

Zur Weiterverwendung des CDENs soll der Umsatzgrad des CDD und des CDT möglichst höher als 99,0% sein, da doppelt ungesättigte Verbindungen wie CDD zur Bildung höhersiedender Fraktionen bei nachfolgenden Reaktionen wie Hydroformulierungen oder Hydratisierungen führen. Die Selektivität bezüglich CDEN sollte über 90 % liegen, damit in CDEN-Folgereaktionen die Raum-Zeit-Ausbeute nicht durch die gebildete inerte Menge CDAN verringert wird.

Eine Möglichkeit der Erzielung einer hohen Ausbeute an CDEN ist die homogene Selektivhydrierung mit Ru-Komplexen. In der US 5 180 870 wird die homogene Hydrierung von CDT mit Ru-Komplexen beschrieben. Dabei werden sterisch gehinderte Amine und freies Triarylphosphin zugesetzt, um den Katalysator partiell zu vergiften. Die Abtrennung des CDENs vom Katalysator und den Aminen ist schwierig und führt zu potentieller Kontamination des Produktes CDEN mit Aminen. In den Patentschriften US 5 177 278, US 3 804 914, US 5 210 349, US 5 128 296 werden Lösungsmittel zur homogenen CDT-Hydrierung mit Ru-Komplexen zugesetzt. Dies führt zu geringen Raum-Zeit-Ausbeuten und Problemen der Trennung von Katalysator und Lösungsmittel vom Reaktionsprodukt. US 6 194 624 beschreibt die homogene Hydrierung ebenfalls mit einem Ruthenium-Katalysator unter Zusatz von Kohlenmonoxid und Carbonsäuren wie beispielsweise Essigsäure oder Propionsäure. Auch hier ist die Abtrennung des CDENs von dem homogenen Katalysator und der Carbonsäure problematisch.

Auch Verfahren zur heterogen katalysierten Herstellung von CDEN sind in der Literatur beschrieben. Für die heterogene Katalyse sind vor allem Palladiumkatalysatoren geeignet.

Daneben wurden auch Untersuchungen mit aktiviertem Kupfer auf einem oxidischen Träger berichtet. Die in Stud. Surf. Sci. Catal. (1991), 63 (Prep. Catal. V), 95 - 102 von Castro et al. dargestellten Ergebnisse mit Kupfer-Katalysatoren zeigen zwar eine beachtliche Selektivität bezüglich CDEN aber nur eine sehr geringe Aktivität. Des weiteren ist sowohl die Selektivität als auch die Aktivität in erheblichem Maße von der Herstellung und der Güte des KatalysatorSystems abhängig. Daher gestaltet sich ein industrieller Einsatz dieser Katalysatoren sehr schwierig, denn die Reproduzierbarkeit der Ergebnisse ist nicht gegeben.

Bei der heterogenen Herstellung von CDEN sind die Verfahrensvarianten der diskontinuierlichen Hydrierung in flüssiger Phase mit suspendiertem Katalysator, die kontinuierliche Dreiphasen-Hydrierung im Festbett (CDT in der Flüssigphase) und die kontinuierliche Gasphasen-Hydrierung im Festbett (CDT in der Gasphase) möglich.

In den amerikanischen Patentschriften US 3 400 164 und US 3 400 166 sowie in der GB 19680712 wird eine diskontinuierliche Hydrierung in flüssiger Phase mit suspendiertem Katalysator beschrieben und gezeigt, dass mit einem Palladiumkatalysator auf einem Träger (5 % Pd auf Aktivkohle) bei hohem CDT/CDD-Umsatz eine CDEN-Selektivität von etwa 94 % erreicht werden kann. Dabei werden auf 100 g CDT 1,4 g dieses Katalysators angesetzt. Die Reaktion wird bei 160 ˚C durchgeführt. Der Wasserstoffdruck wird bis zu einem Verbrauch von 75 % des gesamten eingesetzten Wasserstoffs auf 2,07 bar, dann bis zu einem Wasserstoffverbrauch von 90 % bei 0, 69 bar und schließlich bei 0,345 bar gehalten. Die Hydrierung ist dann nach etwa 1 h abgeschlossen. Bezogen auf die Masse des eingesetzten Palladiums ergibt sich eine durchschnittlichen Belastung von 71g(CDT)/g(Pd)•h.

Nachteilig an dieser Slurry-Variante eines diskontinuierlichen Verfahrens ist die Abtrennung des Katalysators, insbesondere wenn dieser nach einiger Zeit abgerieben ist. Eine nachfolgende Filtration macht das Verfahren aufwändig und führt zwangsläufig zu Ausbeute-Verlusten.

In den Patentschriften US 3 400 164 und US 3 400 166 wird außerdem darauf hingewiesen, dass bei erhöhter Temperatur > 160˚C vermehrt aromatische Ringverbindungen als Nebenprodukte entstehen. In den Patentschriften US 3 400 165 und DE 1 678 829 (Columbian Carbon Co.) wird beschrieben, dass die Bildung dieser aromatischen Verbindungen vom CDT ausgeht. Es wird deshalb eine diskontinuierliche Verfahrensweise vorgeschlagen, bei der bis zu einem sehr hohen Umsatz des CDT bei 160˚ C gearbeitet wird und der weitere Umsatz des CDD bei einer wesentlich höheren Temperatur erfolgen soll. Diese aromatischen Nebenverbindungen sind schwer abzutrennen und erfordern erhöhten Reinigungsaufwand.

Weiterhin sind diskontinuierliche Verfahrensweisen in der Regel für eine großtechnische Produktion wenig geeignet und nur dann akzeptabel, wenn keine Alternative existiert. Die Umstellung der Hydrierung mit suspendiertem Katalysator auf ein kontinuierliches Verfahren würde auf erhebliche Schwierigkeiten stoßen und darüber hinaus aufwändig sein, da für dieses Reaktionssystem von Folgereaktionen und einem Zwischenprodukt als Zielprodukt die Charakteristik eines idealen Rohrreaktors angestrebt werden muss. Dies würde eine Reaktorkaskade mit einer größeren Zahl von Rührkesseln erfordern. Nach jedem Rührkessel müsste der Katalysator vom abfließenden Produkt abgetrennt und in den Rührkessel zurückgeführt werden. Daher ist eine solche Verfahrensweise in der Technik nur schwer durchführbar.

Alternative Verfahrensweisen wären der Rieselbett-Reaktor, bei dem CDT in flüssiger Form gemeinsam mit Wasserstoff über ein Festbett von mit Palladium belegten Pellets geführt wird und ein Festbett-Verfahren mit Flüssigkeits- und Gas-Aufgabe von unten. Versuche mit derartigen Reaktoren/Verfahrensweisen sind in Catal. Today (F. Stueber et al., 1995, 24(1-2), 95 - 101) und in Chemical Engineering Science (R.V. Chaudhari, 2001, 65(2), 557 - 564) beschrieben. Die in Catal. Today beschriebenen Versuche wurden mit einem Schalenkatalysator mit einer Dicke der mit Palladium belegten Schalen von 240 $\mu$m und einem Wasserstoffdruck von 1,5 - 12 bar gearbeitet. Bei Aufgabe der Reaktanden von unten wurde nur eine CDEN-Ausbeute von 70 % bei einem (CDT+CDD) Umsetzungsgrad von 85 % erreicht. Hierbei stellt sich heraus, dass die Aktivitätsprobleme aufgrund von Diffusions- und Transportphänomenen auftreten, die durch die Wahl eines Dreiphasensystems fest/flüssig/gasförmig begründet sind. Die Autoren haben den flüssig/fest-Massentransport als limitierenden Faktor identifiziert.

Des weiteren zeigen die hier beschriebenen Versuche mit Festbett- bzw. Rieselbett-Katalysator eine geringe Selektivität in Bezug auf die simultane Bildung von CDAN, ausgedrückt durch die Tatsache, dass bei dem hier vorgestellten Verfahren im Maximum der CDEN-Bildung schon eine deutliche Bildung von CDAN auftritt.

In dem Artikel in Chemical Engineering Science werden zusätzlich die Betriebsweisen Abstrom und Aufstrom verglichen, wobei für Letztere eine etwas höhere Selektivität festgestellt wird. Der Wasserstoffdruck betrug 12 bar. Die angegebenen Ausbeuten bezüglich CDEN liegen nicht über 35 %. Diese niedrigen Ausbeuten zeigen, dass hier eine technische Realisierung ebenfalls ausgeschlossen ist.

Eine Gasphasenhydrierung von Cyclododecatrien ist aus der Literatur von Wießmeier (Ind. Eng. Chem. Res. (1996), 35(12), 4412 - 4416) bekannt.

Dort wird mittels eines speziell hergestellten monolithischen Mikrostruktur-Reaktors mit Mikroströmungskanälen und gleichmäßigen Mesoporensystemen gearbeitet. Dieser Reaktor wird hergestellt, indem auf strukturiertem Aluminiumdraht durch anodische Oxidation eine gleichmäßige, abhängig von der Elektrolysedauer 20 - 50 $\mu$m dicke oxidische Schicht erzeugt wird. Diese Schicht weist senkrecht zur Oberfläche stehende Poren gleicher Länge (Schichtdicke), gleichen Durchmessers und gleichen Abstandes in einem hexagonalen Muster auf. Die Poren sind an der Grenze Oxid/Aluminium einseitig geschlossen. Die Poren werden mit der Lösung einer Pd-Verbindung mehrfach imprägniert, getrocknet, kalziniert und schließlich reduziert, so dass eine gleichmäßige Belegung dieser Poren mit Pd-Kristalliten (Dispersionsgrad ca. 0,2 - 0,3) entsteht.

Durch Verwendung dieses Reaktors kann ein Umsatzgrad von mehr als 96 % und eine Selektivität bezogen auf Cyclododecen von 88 % erreicht werden. Dabei wurden bereits 8% CDAN gebildet und die Katalysatorbelastung betrug 36 g (CDT)/g(Pd)•h.

Dieses Reaktorsystem hat weiterhin den Nachteil, dass der verwendete Katalysator sehr aufwändig in seiner Herstellung ist und deshalb ein solches System nicht für eine industrielle Herstellungsweise von Cyclododecen geeignet ist.

Darüber hinaus ist in diesem Artikel beschrieben, dass handelsübliche Katalysatoren, die aus einem Trägermaterial mit darin feinst verteilten Edelmetallen bestehen, nicht für die Gasphasenhydrierung von Cyclododecatrien verwendet werden können. Denn aufgrund der ungleichmäßigen Verteilung der Poren auf der Oberfläche dieser herkömmlichen Träger und der damit verbundenen Ungleichmäßigkeit der aktiven Metall-Spezies an der Oberfläche werden die in dem Artikel vordergründig für die schlechte Aktivität des Katalysators verantwortlich gefundenen Probleme mit dem Einfluss des Massentransports immer größer, so dass von den Autoren Wießmeier et al. ausgeschlossen wird, dass handelsübliche Katalysatoren industriell anwendbar sind.

Es bestand daher die Aufgabe, ein Gasphasenverfahren zur selektiven heterogenen Hydrierung von Cyclododecatrien und/oder Cyclododecadien (Edukt) zu Cyclododecen zu finden, das vorzugsweise durch eine kontinuierliche Betriebsweise, eine hohe CDEN-Selektivität bei einem Umsetzungsgrad (CDT + CDD) von über 99,5 % ausgezeichnet ist und im technischen Maßstab zu realisieren ist, somit von den Nachteilen der bisher beschriebenen Verfahren frei ist und einen leicht verfügbaren Katalysator benutzt.

Diese Aufgabe wird erfindungsgemäß gelöst durch ein Verfahren zur Herstellung von Cyclododecen durch selektive Gasphasenhydrierung mindestens eines Eduktes, ausgewählt aus der Gruppe von Cyclododecatrien, Cyclododecadien und Mischungen daraus, in einem Festbettreaktor, dadurch gekennzeichnet, dass die Bodensteinzahl für das Verfahren in dem Festbettreaktor größer ist als 100.

Überraschenderweise wurde gefunden, dass mit einem Katalysator bei Einhaltung sehr geringer Wasserstoffdrucke ein industrielles Verfahren zur heterogenen Gasphasenhydrierung von CDT und/oder CDD zu CDEN gemäß der vorliegenden Erfindung realisiert werden kann. Trotz intensiver Forschungen war ein solches Verfahren bisher nicht bekannt. In Ind. Eng. Chem. Res. (1996), 35 (12), 4412 - 4416 wurde sogar nahe gelegt, dass spezielle mikrostrukturierte Katalysatoren erforderlich sind, um eine hohe CDEN-Selektivität zu erreichen. Dies wird durch die Erfindung als Vorurteil der Technik widerlegt.

Neben Cyclododecatrien können auch weitere cyclische, nicht aromatische, mehrfach ungesättigte Kohlenwasserstoffe, wie zum Beispiel Cyclooctadien oder Trimethylcyclododecatrien, durch das erfindungsgemäße Verfahren selektiv hydriert werden.

Die Bodensteinzahl ist eine reaktionskinetische Kennzahl für die Verweilzeit-Verteilung in einem realen Strömungsrohr nach dem Dispersionsmodell. Sie ist ein Maß für die Rückvermischung. Darüber hinaus wird die Reynoldszahl oft als eine weitere Kennzahl für ein katalytisches Verfahren angegeben. Sie gibt den Strömungszustand wieder. Eine Definition der Bodensteinzahl und der Reynoldszahl finden sich in der Fachliteratur, so zum Beispiel in dem Buch "Chemische Reaktionskinetik" von Baerns et al., Thieme Verlag, 1987. Die Bodensteinzahl ist danach definiert als das Produkt aus mittlerer Strömungsgeschwindigkeit $\bar{u}$ und der Länge des Strömungsrohrs L in Verhältnis zu dem axialen Diffusionskoeffizienten $D_{ax}$.

$$Bo = \frac{\bar{u}\,L}{D_{ax}} = Pe_{ax} \cdot \frac{L}{d_R}$$

Pe = Pecletzahl

Gemäß einer bevorzugten Ausführungsform ist die Bodensteinzahl für das erfindungsgemäße Verfahren größer als 500 und insbesondere größer als 1000.

Bei Einhaltung dieser Parameter für die heterogene Reaktion von gasförmigem Cyclododecatrien und/oder CDD mit Wasserstoff unter Feststoff-Katalyse lassen sich nochmals verbesserte Selektivitäten für die Bildung von Cyclododecen erzielen.

Die Reynoldszahl für das erfindungsgemäße Verfahren ist vorzugsweise größer als 10. Bevorzugt ist die Reynoldszahl größer als 100 und besonders bevorzugt ist sie größer als 200. Auf diese Weise kann eine verbesserte CDEN-Selektivität (mehr als 90%) erzielt werden.

Entsprechend einer weiteren, bevorzugten erfindungsgemäßen Form der vorliegenden Erfindung wird das in der Gasphase vorliegende Ausgangsprodukt in Gegenwart eines Formkörpers hydriert.

Ein solcher Formkörper kann bevorzugt mehr als 90 Gew.-% an Trägermaterial enthalten, bezogen auf das Gesamtgewicht des Formkörpers.

Auf diese Weise ist es möglich, Katalysatoren für das erfindungsgemäße, heterogene Verfahren zu verwenden, die weniger aufwändig in ihrer Herstellung sind und darüber hinaus aufgrund ihres Aufbaus auch gewährleisten, dass ein industrieller Einsatz realisiert werden kann.

Bei dem in dem erfindungsgemäßen Verfahren verwendeten Katalysator handelt es sich um einen Trägerkatalysator, dessen wirksame Komponente ein Metall der VIII. Nebengruppe des Periodischen Systems der Elemente, vorzugsweise Palladium, ist. Als Träger können vorzugsweise Aluminiumoxid, Siliziumoxid, Titandioxid und Zirkondioxid und vorzugsweise γ-Aluminiumoxid dienen. In einem Verfahren gemäß der vorliegenden Erfindung sind Schalenkatalysatoren besonders bevorzugt, in denen die Verteilung des katalytisch aktiven Metalls im Formkörper nicht homogen ist und das Trägermaterial nicht auf Metall aufgebracht ist. Solche Katalysatoren können durch Imprägnierung des äußeren Bereichs des Trägerpellets mit dem Metall oder durch Beschichtung eines dichten Kerns zum Beispiel aus Aluminiumoxid und Imprägnierung dieser Schicht mit dem Metall hergestellt werden. Der Palladiumgehalt beträgt 0,05 bis 5,0 Massenprozent bezogen auf das gesamte Katalysatorpellet, vorzugsweise 0,5 bis 1,0 Massenprozent. Die Dicke der äußeren Schicht des Katalysator-Formkörpers beträgt dabei höchstens 1/10 der maximalen Abmessung des Formkörpers und in dieser äußeren Schicht ist mehr als 70 Gew.-% des aktiven Metalls des Formkörpers enthalten.

In einer weiteren bevorzugten Ausführungsform der vorliegenden Erfindung ist der Formkörper im wesentlichen rund, insbesondere ist er kugelförmig.

Aufgrund dieser Geometrie erhält man ein effektives Verhältnis zwischen der Masse des einzusetzenden Katalysators und seiner mit aktivem Metall beschickten Oberfläche.

Der Formkörper hat einen Durchmesser von mehr als 0,5 mm, insbesondere von mehr als 2 mm. In der Regel liegen die Formkörper in einem Festbett vor.

Diese Reaktionsführung ermöglicht eine leichte Abtrennung von Produkt und Katalysator.

Die Belastung des in dem erfindungsgemäßen Verfahren eingesetzten Katalysators ist definiert als Masse Edukt/Masse Pd•h. Sie beträgt vorzugsweise 15 bis 500, besonders bevorzugt 20 bis 100 g Edukt/g Pd•h und ist neben den Kennzahlen Bodensteinzahl und Reynoldszahl eine wichtige Verfahrensvariable.

Im erfindungsgemäßen Verfahren wird das Edukt einem Kreisgasstrom verdampft. Dieses Kreisgas besteht im Wesentlichen aus Inertgas wie Stickstoff, Methan, Kohlendioxid, Helium, Neon, Argon, Krypton, Xenon oder Mischungen dieser Gase, vorzugsweise aus Stickstoff. Der Partialdruck des CDT im Kreisgasstrom ist so einzustellen, dass er unter dem Sättigungsdampfdruck des CDT bei Reaktionstemperatur liegt, vorzugsweise bei 50 bis 80 % des Sättigungsdruckes. Der Partialdruck des Wasserstoffs im Kreisgas ist erfindungsgemäß so einzustellen, dass sich ein Umsatz (CDT+CDD) von mindestens 99 % ergibt. Im stationären Betriebszustand der Anlage wird der Wasserstoff gemäß Verbrauch nachdosiert. Der Gesamtdruck des Kreisgases ist für die Verfahrensführung unkritisch und beträgt vorzugsweise 50 bis 10000 kPa.

Um die CDEN-Selektivität zu steigern, kann die Wasserstoffzufuhr so aufgeteilt werden, dass nur ein Teil des Wasserstoffs am Reaktoreingang und die restliche Menge über den Reaktor verteilt eingespeist wird. Vorzugsweise werden zwei Drittel der Gesamtmenge an Wasserstoff am Reaktoreingang und der Rest an dem Ort des Reaktors eingespeist, an dem CDT weitgehend umgesetzt ist. Insgesamt beträgt die molare Menge an Wasserstoff vorzugsweise zwischen dem 0,9- und 1,2-fachen der theoretischen Menge liegen, die benötigt wird um das Ausgangsprodukt vorhandene Cyclododecatrien und/oder Cyclododecadien zum Cyclododecen zu hydrieren.

Eine weitere Steigerung der Selektivität zu CDEN kann erreicht werden, wenn als Reaktionsgas Kohlenmonoxid zugeführt wird. Der Partialdruck des Kohlenmonoxids, gemessen am Reaktorausgang, kann in den Grenzen von 10 bis 1 000 Pa variiert werden.

Die Reaktionstemperatur kann in weiten Grenzen variiert werden. Bevorzugt ist eine Reaktionstemperatur von 90 bis 180˚ C, besonders bevorzugt ist der Bereich von 100 bis 160˚ C. Die Temperatur des Reaktors muss nicht konstant sein, sondern kann in den oben genannten Grenzen variiert werden. Dabei ist es möglich, ein ansteigendes oder abfallendes Temperaturprofil im Reaktor einzustellen.

Als Reaktortyp kann ein Schüttbett-Reaktor gewählt werden, dessen Durchmesser/Längen-Verhältnis so gewählt ist, dass eine weitgehende Rohrreaktor-Charakteristik und damit eine geringere Rückvermischung erreicht wird. Vorzugsweise beträgt dessen Durchmesser/Längen-Verhältnis 0,03 bis 0,38. Als weiterer Reaktortyp ist ein Rohrbündelreaktor geeignet, ohne die Erfindung auf diese Reaktortypen zu beschränken.

In einer besonders bevorzugten Ausführungsform der vorliegenden Erfindung wird das Verfahren zur selektiven Hydrierung von Cyclododecatrien und/oder Cyclododecadien kontinuierlich betrieben.

### Beispiel 1 (nicht erfindungsgemäß):

Dieses Beispiel wurde aus der Dissertation von Dipl. Ing. Georg F. L. Wießmeier mit dem Titel: "Monolithische Mikrostruktur-Reaktoren mit Mikroströmungskanälen und regelmäßigen Mesoporensystemen für selektive, heterogen katalysierte Gasphasenreaktionen" Shaker Verlag 1997, ISBN 3-8265-2183-8 entnommen.

In einem röhrenförmigen Reaktor mit einem Durchmesser von 8,5 mm wurden 0,16 g des Katalysators Heraeus K-0240 (0,5 % Pd auf Aluminiumoxid), der auf eine mittlere Korngröße von 400 $\mu$m zerkleinert wurden war, eingefüllt. Mit einer Schüttdichte von etwa 1 g/cm$^3$ ergab sich eine Schütthöhe von 2,8 mm. Es wurde nun ein Volumenstrom von 8,6 1/h eingestellt, woraus sich eine Strömungsgeschwindigkeit von 0,04m/s ergab. Der Gesamtdruck betrug 110 kPa, der Cyclododecatrien (CDT)-Druck 110 Pa, der Wasserstoffdruck 330 Pa (Rest Stickstoff) und die Reaktionstemperatur 120 ˚C. Aus diesen Daten ergibt sich nach Rechnung eine partikelbezogene Reynoldszahl $Re_p$ = 0,7 und mit einer axialen partikelbezogenen Pecletzahl $Pe_{ax,p}$ = 2 eine Bodensteinzahl Bo = 14. Damit wurde eine Selektivität von maximal 62 % CDEN bei einem CDT-Umsatz von 80% erreicht. Damit ist gezeigt, dass bei geringen Bodensteinzahlen, was hohe Rückvermischung heißt, nur geringe Selektivitäten in der Selektivhydrierung von Cyclododecatrien zu Cyclododecen erreicht werden können.

### Beispiel 2 (erfindungsgemäß):

Ein Schalenkatalysator mit 0,5 % Pd auf Aluminiumoxid und einem mittleren Teilchendurchmesser von 2,5 mm wurde in einer 2 m hohen Schüttung in einen Rohrreaktor von 1,46 m$^2$ Querschnittfläche eingefüllt. Mit einer Strömungsgeschwindigkeit von 1,58 m/s ergibt sich eine partikelbezogene Reynoldszahl $Re_p$ = 247 und mit einer axialen partikelbezogenen Pecletzahl $Pe_{ax,p}$ = 2 eine Bodensteinzahl Bo = 1600. Hiermit wurde bei 120 ˚C und ansonsten gleichen Druck und Konzentrationsverhältnissen wie in Beispiel 1 eine Selektivität von 90 % CDEN bei einem CDT-Umsatz von 100 % erreicht. An restlichem CDD verblieben 0,2%. Somit betrug der Gesamtumsatz (CDT + CDD) 99,8%.

Damit ist gezeigt, dass bei hohen Bodensteinzahlen, was geringe Rückvermischung heißt, deutlich höhere Selektivitäten in der Selektivhydrierung von Cyclododecatrien zu Cyclododecen erreicht werden können.

### Beispiel 3 (erfindungsgemäß):

Es wurde analog Beispiel 2 verfahren, aber dem Reaktionsgas wurden noch 3000 ppm Kohlenmonoxid beigemischt. Hiermit wurde eine Selektivität von 94 % CDEN bei einem CDT-Umsatz von 100 % erreicht. An restlichem CDD verblieben 0,2 %. Somit betrug der Gesamtumsatz (CDT + CDD) 99,8%.

Anhand dieses Beispiels wird deutlich, dass durch den Zusatz von Kohlenmonoxid zum Reaktionsgas eine nochmalige Steigerung in der Selektivität erzielt wird..

**Patentansprüche**

1. Verfahren zur Herstellung von Cyclododecen durch selektive Gasphasenhydrierung mindestens eines Ausgangsprodukts, ausgewählt aus der Gruppe von Cyclododecatrien, Cyclododecadien und Mischungen daraus, in einem Festbettreaktor,
   **dadurch gekennzeichnet,**
   **dass** die Bodensteinzahl in dem Festbettreaktor größer ist als 100.

2. Verfahren nach Anspruch 1,
   **dadurch gekennzeichnet,**
   **dass** die Bodensteinzahl für das Verfahren in dem Festbettreaktor größer als 500, insbesondere größer als 1000, ist.

3. Verfahren nach Anspruch 1 oder 2,
   **dadurch gekennzeichnet,**
   **dass** die Reynoldszahl größer als 10, insbesondere größer als 100 und besonders bevorzugt größer als 200 ist.

4. Verfahren nach einem der vorhergehenden Ansprüche,
   **dadurch gekennzeichnet,**
   **dass** die Belastung des Katalysators 15 bis 500 vorzugsweise 20 bis 100 g Cyclododecatrien und/oder Cyclododecadien / g Pd•h beträgt.

5. Verfahren nach einem der vorhergehenden Ansprüche,
   **dadurch gekennzeichnet,**
   **dass** das in Gasphase vorliegende Ausgangsprodukt in Gegenwart eines Formkörpers hydriert wird.

6. Verfahren nach einem der vorhergehenden Ansprüche,
   **dadurch gekennzeichnet,**
   **dass** der Formkörper mehr als 90 Gew.-% an Trägermaterial enthält, bezogen auf den Formkörper.

7. Verfahren nach einem der vorhergehenden Ansprüche,
   **dadurch gekennzeichnet,**
   **dass** der Formkörper im wesentlichen rund ist und insbesondere kugelförmig ist.

8. Verfahren nach einem der vorhergehenden Ansprüche,
   **dadurch gekennzeichnet,**
   **dass** der Durchmesser des Formkörpers mehr als 0,5 mm, insbesondere mehr als 2 mm, beträgt.

9. Verfahren nach einem der vorhergehenden Ansprüche,
   **dadurch gekennzeichnet,**
   **dass** das Trägermaterial des Formkörpers $\gamma$-Aluminiumoxid ist.

10. Verfahren nach einem der vorhergehenden Ansprüche,
    **dadurch gekennzeichnet,**
    **dass** das Trägermaterial nicht auf Metall aufgebracht ist.

11. Verfahren nach einem der vorhergehenden Ansprüche,
    **dadurch gekennzeichnet,**
    **dass** der Formkörper mindestens ein fein verteiltes, katalytisch aktives Metall der Gruppe VIII des Periodischen Systems der Elemente enthält.

12. Verfahren nach einem der vorhergehenden Ansprüche,
    **dadurch gekennzeichnet,**
    **dass** die Verteilung des katalytisch aktiven Metalls im Formkörper nicht homogen ist.

**13.** Heterogenes Verfahren nach einem der vorhergehenden Ansprüche,
**dadurch gekennzeichnet,**
**dass** der Formkörper eine äußere Schicht aufweist;

• die Dicke der äußeren Schicht höchstens 1/10 der maximalen Abmessung des Formkörpers beträgt; und
• in dieser Schicht mehr als 70 Gew.-% des katalytisch aktiven Metalls des Formkörpers vorhanden ist.

**14.** Verfahren nach einem der vorhergehenden Ansprüche,
**dadurch gekennzeichnet,**
**dass** das katalytisch aktive Material Palladium ist.

**15.** Verfahren nach einem der vorhergehenden Ansprüche,
**dadurch gekennzeichnet,**
**dass** die molare Menge an Wasserstoff zwischen dem 0,9- und 1,2-fachen der theoretischen Menge liegt, die benötigt wird, um das im Ausgangsprodukt vorhandene Cyclododecatrien und/oder Cyclododecadien zum Cyclododecen zu hydrieren.

**16.** Verfahren nach einem der vorhergehenden Ansprüche,
**dadurch gekennzeichnet,**
**dass** bei einer Temperatur zwischen 90 und 180 ˚C, bevorzugt zwischen 100 und 160 ˚C, hydriert wird.

**17.** Verfahren nach einem der vorhergehenden Ansprüche,
**dadurch gekennzeichnet,**
**dass** der Gasphase zusätzlich Kohlenmonoxid zugeführt wird.

**18.** Verfahren nach einem der vorhergehenden Ansprüche,
**dadurch gekennzeichnet,**
**dass** die Gasphase zusätzlich ein Inertgas, insbesondere Stickstoff, enthält.

**19.** Verfahren nach einem der vorhergehenden Ansprüche,
**dadurch gekennzeichnet,**
**dass** das Ausgangsprodukt in Inertgasatmosphäre verdampft wird.

**20.** Verfahren nach einem der vorhergehenden Ansprüche,
**dadurch gekennzeichnet,**
**dass** der Gesamtdruck der Gasphase zwischen 50 und 10.000 hPa beträgt.

**21.** Verfahren nach einem der vorhergehenden Ansprüche,
**dadurch gekennzeichnet,**
**dass** es kontinuierlich betrieben wird.

## Claims

**1.** Process for preparing cyclododecene by selective gas-phase hydrogenation of at least one starting material selected from the group consisting of cyclododecatriene, cyclododecadiene and mixtures thereof in a fixed-bed reactor,
**characterized in that**,
the Bodenstein number in the fixed-bed reactor is greater than 100.

**2.** Process according to Claim 1,
**characterized in that**
the Bodenstein number for the process in the fixed-bed reactor is greater than 500, in particular greater than 1000.

**3.** Process according to Claim 1 or 2,
**characterized in that**
the Reynolds number is greater than 10, in particular greater than 100 and particularly preferably greater than 200.

**4.** Process according to any of the preceding claims,

**characterized in that**

the throughput per amount of catalyst is from 15 to 500 g, preferably from 20 to 100 g, of cyclododecatriene and/or cyclododecadiene/g Pd•h.

5. Process according to any of the preceding claims,
   **characterized in that**
   the starting material present in the gas phase is hydrogenated in the presence of a shaped body.

6. Process according to any of the preceding claims,
   **characterized in that**
   the shaped body comprises more than 90% by weight of support material, based on the shaped body.

7. Process according to any of the preceding claims,
   **characterized in that**
   the shaped body is essentially round and in particular is spherical.

8. Process according to any of the preceding claims,
   **characterized in that**
   the diameter of the shaped body is more than 0.5 mm, in particular more than 2 mm.

9. Process according to any of the preceding claims,
   **characterized in that**
   the support material of the shaped body is γ-aluminum oxide.

10. Process according to any of the preceding claims,
    **characterized in that**
    the support material has not been applied to metal.

11. Process according to any of the preceding claims,
    **characterized in that**
    the shaped body comprises at least one finely divided, catalytically active metal of group VIII of the Periodic Table of the Elements.

12. Process according to any of the preceding claims,
    **characterized in that**
    the distribution of the catalytically active metal in the shaped body is not homogeneous.

13. Heterogeneous process according to any of the preceding claims,
    **characterized in that**
    the shaped body has an outer layer;

    • the thickness of the outer layer is not more than 1/10 of the maximum dimension of the shaped body; and
    • more than 70% by weight of the catalytically active metal of the shaped body is present in this layer.

14. Process according to any of the preceding claims,
    **characterized in that**
    the catalytically active material is palladium.

15. Process according to any of the preceding claims,
    **characterized in that**
    the molar amount of hydrogen is from 0.9 to 1.2 times the amount which is theoretically required to hydrogenate the cyclododecatriene and/or cyclododecadiene present in the starting material to cyclododecene.

16. Process according to any of the preceding claims,
    **characterized in that**
    the hydrogenation is carried out at a temperature in the range from 90 to 180°C, preferably from 100 to 160°C.

17. Process according to any of the preceding claims,

**characterized in that**
carbon monoxide is additionally introduced into the gas phase.

18. Process according to any of the preceding claims,
**characterized in that**
the gas phase further comprises an inert gas, in particular nitrogen.

19. Process according to any of the preceding claims,
**characterized in that**
the starting material is vaporized in an inert gas atmosphere.

20. Process according to any of the preceding claims,
**characterized in that**
the total pressure of the gas phase is from 50 to 10 000 hPa.

21. Process according to any of the preceding claims **characterized in that** it is carried out continuously.

**Revendications**

1. Procédé en vue de la fabrication de cyclododécène par hydrogénation sélective en phase gazeuse d'au moins un produit de départ, sélectionné parmi le groupe du cyclododécatriène, du cyclododécadiène et de mélanges de ces derniers, dans un réacteur à lit fixe, **caractérisé en ce que** l'indice de Bodenstein dans le réacteur à lit fixe est supérieur à 100.

2. Procédé selon la revendication 1, **caractérisé en ce que** l'indice de Bodenstein pour le procédé dans le réacteur à lit fixe est supérieur à 500, en particulier, supérieur à 1000.

3. Procédé selon la revendication 1 ou 2, **caractérisé en ce que** l'indice de Reynolds est supérieur à 10, en particulier supérieur à 100 et, en particulier, de préférence, supérieur à 200.

4. Procédé selon l'une quelconque des revendications précédentes, **caractérisé en ce que** la charge du catalyseur est de 15 à 500, de préférence, de 20 à 100 g de cyclododécatriène et/ou de cyclododécadiène / g Pd*h.

5. Procédé selon l'une quelconque des revendications précédentes, **caractérisé en ce que** le produit de départ présent dans la phase gazeuse est hydrogéné en présence d'un corps moulé.

6. Procédé selon l'une quelconque des revendications précédentes, **caractérisé en ce que** le corps moulé contient plus de 90% en poids de matériau de support, par rapport au corps moulé.

7. Procédé selon l'une quelconque des revendications précédentes, **caractérisé en ce que** le corps moulé est, pour l'essentiel, rond et, en particulier, sous forme de bille.

8. Procédé selon l'une quelconque des revendications précédentes, **caractérisé en ce que** le diamètre du corps moulé est supérieur à 0,5 mm, en particulier, supérieur à 2 mm.

9. Procédé selon l'une quelconque des revendications précédentes, **caractérisé en ce que** le matériau de support du corps moulé est l'oxyde de γ-aluminium.

10. Procédé selon l'une quelconque des revendications précédentes, **caractérisé en ce que** le matériau de support n'est pas appliqué sur le métal.

11. Procédé selon l'une quelconque des revendications précédentes, **caractérisé en ce que** le corps moulé contient au moins un métal actif du point de vue catalytique, finement divisé, du groupe VIII du système périodique des éléments.

12. Procédé selon l'une quelconque des revendications précédentes, **caractérisé en ce que** la répartition du métal actif du point de vue catalytique dans le corps moulé n'est pas homogène.

**13.** Procédé hétérogène selon l'une quelconque des revendications précédentes, **caractérisé en ce que** le corps moulé présente une couche externe;

    • l'épaisseur de la couche externe étant d'au plus 1/10 de la dimension maximale du corps moulé; et
    • plus de 70% en poids du métal actif du point de vue catalytique du corps moulé étant présents dans cette couche.

**14.** Procédé selon l'une quelconque des revendications précédentes, **caractérisé en ce que** le matériau actif du point de vue catalytique est le palladium.

**15.** Procédé selon l'une quelconque des revendications précédentes, **caractérisé en ce que** la quantité molaire d'hydrogène se situe entre 0,9 et 1,2 fois de la quantité théorique, qui est nécessaire pour hydrogéner le cyclododécatriène et/ou le cyclododécadiène pour former du cyclododécène.

**16.** Procédé selon l'une quelconque des revendications précédentes, **caractérisé en ce que** l'on procède à l'hydrogénation à une température comprise entre 90 et 180˚C, de préférence, entre 100 et 160˚C.

**17.** Procédé selon l'une quelconque des revendications précédentes, **caractérisé en ce que** l'on alimente à la phase gazeuse en sus du monoxyde de carbone.

**18.** Procédé selon l'une quelconque des revendications précédentes, **caractérisé en ce que** la phase gazeuse contient en outre un gaz inerte, en particulier l'azote.

**19.** Procédé selon l'une quelconque des revendications précédentes, **caractérisé en ce que** le produit de départ est évaporé sous une atmosphère inerte.

**20.** Procédé selon l'une quelconque des revendications précédentes, **caractérisé en ce que** la pression globale de la phase gazeuse est comprise entre 50 et 10000 hPa.

**21.** Procédé selon l'une quelconque des revendications précédentes, **caractérisé en ce qu'**il fonctionne en mode continu.